# EUROPEAN PATENT SPECIFICATION

(11) **EP 4 486 731 B1**
(45) Date of publication and mention of the grant of the patent: **05.08.2026**
(21) Application number: 23713172.7
(22) Date of filing: 28.02.2023
(51) Int. Cl.: C07D 307/46

(54) **SYNTHESIS OF 5-ACETOXYMETHYL FURFURAL IN THE PRESENCE OF RECYCLABLE SALT CATALYSTS**
SYNTHESE VON 5-ACETOXYMETHYLFURFURAL IN GEGENWART VON WIEDERVERWENDBAREN SALZKATALYSATOREN
SYNTHÈSE DE 5-ACÉTOXYMÉTHYLFURFURAL EN PRÉSENCE DE CATALYSEURS SALINS RECYCLABLES

(30) Priority: 01.03.2022 US 202263315220 P
(43) Date of publication of application: 08.01.2025
(73) Proprietor: Celluflux Ltd., 9371536 Jerusalem (IL)
(72) Inventor: MELLER, Elad, 9371536 Jerusalem (IL); LEADER, Avia, 9330215 Jerusalem (IL)
(74) Representative: Walter, Kai U.
(86) International application number: PCT/IL2023/050207
(87) International publication number: WO 2023/166503

(56) References cited:
- WO-A1-2011/043661
- WO-A1-2017/076942
- US-A1- 2008 200 698
- FAYET CATHERINE ET AL: "NOUVELLE MÉTHODE DE PRÉPARATION DU 5-HYDROXYMÉTHYL- 2-FURALDÉHYDE PAR ACTION DE SELS D'AMMONIUM OU D'IMMONIUM SUR LES MONO-. OLIGO- ET POLY-SACCHARIDES. ACCÈS DIRECT AUX 5-HALOGÉNOMÉTHYL-2-FURALDÉHYDES*", CARBOHYDRATES RESEARCH, 122 (1983), 1 January 1983 (1983-01-01), pages 59 - 68, XP093050481, Retrieved from the Internet <URL:https://www.sciencedirect.com/journal/carbohydrate-research/vol/122/issue/1> [retrieved on 20230530]

## Description

### Background of the invention

Carbohydrates are a renewable resource that can replace petroleum to produce chemicals, fuels, and fuel additives. The carbohydrates-derived chemicals industry has grown considerably in recent years and is expected to keep growing in the future.

Carbohydrates are subdivided into simple monosaccharides, such as fructose, galactose, or glucose, disaccharides which are formed from two monosaccharides, e.g., sucrose, lactose, or maltose. Carbohydrates formed from 3 to about 10 monosaccharides are called oligosaccharides, e.g., raffinose, stachyose, or dextrins, and those containing more than 10 monosaccharides are called polysaccharides, e.g., glycogen, starch, or cellulose.

Another important type of monosaccharides classification is the number of carbon atoms in the sugar molecule. According to the number of carbon atoms in the molecule, monosaccharides are subdivided into triose (3 carbon atoms), tetrose (4 carbon atoms), pentose (5 carbon atoms), hexose (6 carbon atoms), or heptose (7 carbon atoms). Hexoses that are based on the furan five-membered ring are often called furanoses (or furanoids). Likewise, hexoses are classified as pyranoses (or pyranoids) if they are based on the six-membered pyran ring. 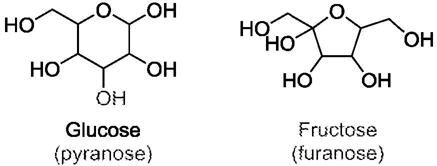 (no stereochemistry is shown)

It should also be noted that monosaccharides exist in equilibrium between their open-chain and various closed-chain forms; this equilibrium is called "ring-chain tautomerism". For example, the ring-chain tautomerism for D-fructose is shown below: 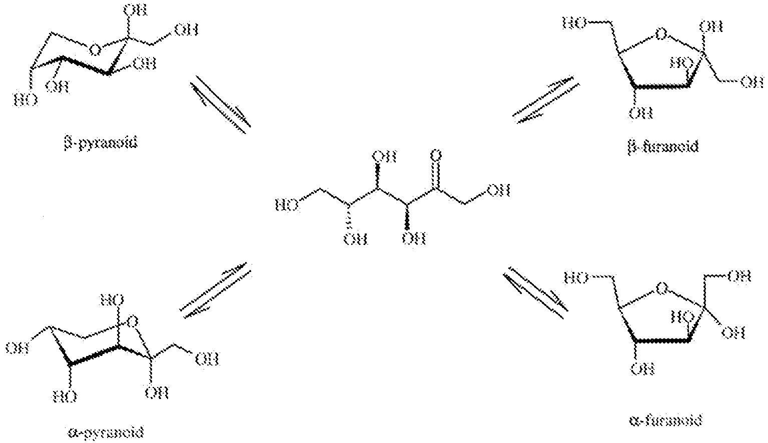 (see for example M. Bicker et al., "Dehydration of d-fructose to hydroxymethylfurfural in sub- and supercritical fluids", J. of Supercritical Fluids, 36, 2005, 118-126).

One of the important carbohydrate derivatives is 5-Hydroxymethylfurfural (HMF) which was identified as one of the most promising bio-based chemicals (see J.J. Bozell, G.R. Petersen, "Technology development for the production of biobased products from biorefinery carbohydrates - the US Department of Energy's "Top 10" revisited", Green Chem., 12, 2010, 539-554).

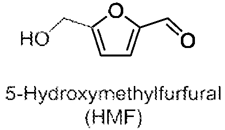

HMF can be used as a platform for the production of diverse highly valuable chemical products with significant economic importance such as fuels, monomers, resins, surfactants, solvents, etc.

HMF can be produced from monosaccharides like fructose or glucose, or di-, oligo- and polysaccharides that contain C₆ sugar units such as sucrose, starch, cellulose, etc. HMF production process proceeds via dehydration, most occasionally with acidic catalysts. Generally, the process is more efficiently carried out with fructose as a starting material. The process starting from glucose requires an additional isomerization step while reaction starting from oligo or polysaccharides needs a depolymerization step which results in lower yield.

Transforming carbohydrates to HMF in an aqueous medium brings poor yields due to the instability of HMF in the aqueous medium, especially in the presence of an acidic catalyst, and the formation of by-products such as levulinic acid and formic acid via hydrolysis and humic substances via polymerization and condensation.

Since by-products formation is favored in an aqueous medium, other solvents for HMF preparation were studied. Organic polar aprotic solvents, including DMSO, DMF, DMA, NMP, etc., are more efficient than water as a reaction medium since the hydrolysis of HMF is inhibited. For example, up to 97% yield of HMF from fructose was demonstrated with DMSO as a solvent of reaction in US 4,590,283.

Although higher yields of HMF and a significant reduction in side-products formation can be achieved with high boiling polar aprotic solvents, and particularly with DMSO, the recovery of HMF from such solvents is a major challenge in large scale production. In another approach, HMF preparation is carried out in a biphasic system consisting of an aqueous phase and an organic phase. In this methodology, HMF is extracted in-situ from the aqueous phase by the organic solvent, thereby side reactions are minimized. For example, Dumesic et al. reported a biphasic system of MIBK as an organic solvent and an aqueous phase with hydrochloric acid or acidic ion-exchange resin as catalysts for the transformation of fructose into HMF. In this system, 80% HMF selectivity at 90% fructose conversion was achieved (Y. Roman-Leshkov, J.N. Chheda, J.A. Dumesic, "Phase modifiers promote efficient production of hydroxymethylfurfural from fructose", Science, 312, 2006, 1933-1937). However, due to the complexity of a multi-solvent plant design, the economical attractiveness of the process is low.

High yields of HMF can be achieved in ionic liquid media. For example, dehydration of fructose in methyl imidazolium chloride afforded a 92% yield of HMF (C. Moreau, A. Finiels and L. Vanoye, J., "Dehydration of fructose and sucrose into 5-hydroxymethylfurfural in the presence of 1-H-3-methyl imidazolium chloride acting both as solvent and catalyst", J. of Mol. Catal. A: Chem., 253, 2006, 165-169).

A different strategy to obtain furanic valuable compounds is to convert carbohydrates into HMF derivatives such as HMF esters. Such ester derivatives are more stable and easier to work-up and isolate compared to HMF.

For example, 5-Acetoxymethyl furfural (AMF), acetyl ester of HMF, is an ecofriendly and, potentially, a cheap ester of HMF. Because of its hydrophobicity, AMF is easier to extract compared to HMF. Furthermore, due to its higher stability, it can be purified by vacuum distillation with only minimal loss of the product (DE 3309564 A1), while distilling the thermally unstable HMF results in a substantial loss of the product due to its polymerization. Moreover, it should be noted that while HMF shows weak cytotoxicity and mutagenicity, AMF is neither cytotoxic nor mutagenic (R.J. van Putten, J.C. van der Waal, E. de Jong, C.B. Rasrendra, H.J. Heeres, J.G. de Vries, "Hydroxymethylfurfural, a versatile platform chemical made from renewable resources", Chem. Rev., 113, 3, 2013, 1499-1597.).

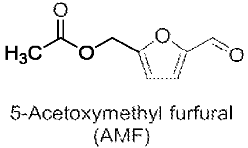

Like HMF, AMF can be utilized as a starting material/intermediate for the preparation of a variety of useful end-products. For example, AMF can be used in the synthesis of bio-monomers such as 2,5-furandicarboxylic acid (FDCA) (US 8,558,018), 2,5-bis(hydroxymethyl)furan and 5-hydroxymethyl-furancarboxylic acid (E.S. Kang et al., "From lignocellulosic biomass to furans via 5-acetoxymethylfurfural as an alternative to 5-hydroxymethyl furfural", ChemSusChem, 8, 2015, 1179-1188). In addition, AMF can be used as a starting material for various amines (A.L. Nuzhdin et al., "Reductive amination of 5-acetoxymethylfurfural over Pt/Al2O3 catalyst in a flow reactor", Mol. Catal., 499, 2021, 111297).

AMF can also find use in resins production, as was demonstrated for the condensation of AMF with urea in the preparation of resins that can be used as binders for foundry molds (US 9,636,742).

In some applications, AMF can be used directly without any modification. For example, due to its high energy content of 8.7 kWh/L, compared to 8.8 kWh/L for gasoline and 6.1 kWh/L for ethanol, AMF can be utilized as a fuel candidate or as a fuel additive alternative to MTBE (US 8,242,293).

WO 2013/121264 discloses AMF as a sweet taste modulator that can enhance the sweetness of reduced-calorie food products.

AMF might also find therapeutic use, for example in inhibiting sickle cell disease showing longer half-live and increased bioavailability compared to HMF (US 10,875,834).

Due to the enormous economical and industrial potential and the significant advantages of HMF esters, and particularly of AMF, over HMF, an industrially practical production process for AMF and other HMF esters from carbohydrates is highly desired. However, relatively little was reported on the transformation of 6 carbon atoms sugars to AMF, or other HMF esters, as compared to their transformation to HMF.

A two-step process for AMF synthesis from carbohydrates via halogenated furfural derivatives, such as 5-chloromethyl furfural (CMF), was demonstrated by E. S. Kang et al. (E.S. Kang et al., "From lignocellulosic biomass to furans via 5-acetoxymethylfurfural as an alternative to 5-hydroxymethylfurfural", ChemSusChem, 8, 2015, 1179-1188). It was shown that CMF, which was formed from carbohydrates, can be converted into AMF in yields of up to 94% by using alkylammonium acetates as catalysts and sodium acetate as acetate source in the acetylation reaction.

A two-step process for AMF production from fructose has been disclosed by Huynh et al. (N.T.T. Huynh, K.W. Lee, J.K. Cho, Y.J. Kim, S.W. Bae, J.S. Shin, S. Shin, "Conversion of D-fructose to 5-acetoxymethyl-2-furfural using immobilized lipase and cation exchange resin", Molecules, 24, 2019, 4623). In the first step, fructose was converted to 1,6-diacetylfructose with immobilized lipase enzymes catalysts and vinyl acetate as acyl donor. In the second step, the acetylated fructose was dehydrated to AMF with acidic ion-exchange resin, amberlyst-15, in DMSO, and 1,4-dioxane.

Furthermore, it is generally preferred to produce AMF directly from carbohydrates rather than in multi-step processes. Several patents and research publications demonstrated a direct route for AMF from hexoses.

The preparation of AMF in sub-critical acetic acid was described by Bicker et al. (M. Bicker et al., "Dehydration of D-fructose to hydroxymethylfurfural in sub- and supercritical fluids", J. of Supercritical Fluids, 36, 2005, 118-126). The reaction was carried out under a pressure of 20MPa and a temperature of 180 °C; these conditions are unacceptable for an industrial-scale process. Furthermore, the authors used sulfuric acid as a catalyst which results in a challenge for catalyst and solvent recovery.

US 8,314,260 discloses the production of HMF ethers and esters (including AMF) directly from carbohydrates in an ionic liquid system with a Lewis acid metal chloride catalyst and an organic acid as an ester-forming agent. Ionic liquids, however, are expensive and difficult to purify and recover, especially from water that is formed in the dehydration reaction.

Conversion of cellulose into AMF in a two-step process was described (L. Gavila, D. Esposito, "Cellulose acetate as a convenient intermediate for the preparation of 5-acetoxymethylfurfural from biomass", Green Chem, 19, 2017, 2496-2500). The first step includes the conversion of cellulose into cellulose acetate, primarily the triacetate form, with acetic anhydride and sulfuric acid as a catalyst. In the second step, the acetylated cellulose was hydrolyzed to AMF in acetic acid as a solvent in the presence of acetic anhydride and a sulfuric acid catalyst. The procedure was tested in batch and continuous flow reactors. A maximum yield of 49% AMF in a flow reactor has been achieved from cellulose acetate with a residence time of 5 minutes. However, in this procedure, only low concentrations of cellulose acetate (5 g/L) proved to be effective. In addition, the recovery of the catalyst and the acetic acid reactive solvent is a major challenge.

The use of chloride salts and acidic ion exchangers for the one-pot, two steps, preparation of AMF from carbohydrates in a temperature range of 80-140 °C was illustrated in US 8,642,790. The preferred catalyst, magnesium chloride hexahydrate, was stirred under heating with the carboxylic acid solvent and a carbohydrate reactant. Then, after removing 80% of the acetic acid, the acetylation was afforded by adding acetic anhydride and a pyridine derivative as a catalyst. After workup, a 45% yield of AMF was demonstrated from fructose.

The direct conversion of mono and disaccharides into AMF with tin montmorillonite (Sn-Mont) as a heterogeneous catalyst was performed utilizing acetic acid as a reaction medium and acetylation agent (S. Shinde, K. Deval, R. Chikate, C. Rode, "Cascade synthesis of 5-(acetoxymethyl)furfural from carbohydrates over Sn-Mont catalyst", ChemistrySelect, 3, 2018, 8770-8778).

Alkali halides catalysts were tested in carboxylic acid ester as a reaction medium for the conversion of carbohydrates in US 10,259,797. It was demonstrated that the formation of the HMF and AMF mixture with subsequent oxidation leads to FDCA, a useful bio-monomer. When using methyl acetate as the reaction medium, methyl acetate is hydrolyzed by water to methanol and acetic acid during the dehydration of the sugar, so that the formation of HMF, and hence AMF, also occurs.

### The invention

We have found that C6 carbohydrates are converted in carboxylic acid(s), in the presence of salts showing low solubility in the carboxylic acid(s), to the corresponding organic acid ester(s) of 5-hydroxymethylfurfural. The reaction affords good selectivity toward the ester formation over 5-hydroxymethylfurfural.

That is, the reaction product consists of a mixture of 5-hydroxymethylfurfural and the corresponding ester(s), e.g., a mixture of AMF and HMF, proportioned in the range from, e.g., 2:3 to 100:1, with AMF preferably being the predominant component of the products mixture, e.g., 1:1 to 100:1, 1:1 to 10:1, 1:1 to 5:1, or even the only detectable product. A higher AMF selectivity can be obtained depending on temperature, catalyst type and reaction time:

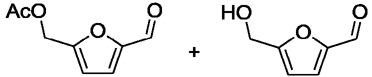

The selection criteria for the salt used to catalyze the reaction is its solubility in the organic acid under consideration. For example, salts that are at most sparingly soluble in the organic acid are used, and even better, salts that are only slightly soluble in the organic acid or practically insoluble. By "sparingly soluble" we mean that not more than ten, less than five, or less than three parts of salt are dissolved in one hundred parts of the organic acid; and by "slightly soluble" we mean that not more than one part of salt is dissolved in one hundred parts of the organic acid, at room temperature. Owing to their fairly low solubility in the reaction medium, the salts are readily separated, e.g., by filtration from the organic acid, and can be recycled for use in a subsequent batch.

Accordingly, the invention is primarily directed to a process for preparing esters of 5-hydroxymethylfurfural of the formula: 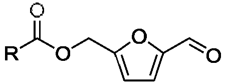 wherein R is a linear or branched alkyl or alkenyl, comprising adding to a reaction vessel C₆ carbohydrate, carboxylic acid R-COOH and a salt which is at most sparingly soluble in a said organic acid to form a reaction mass, wherein the salt is selected such that no more than three parts of the salt are dissolved in one hundred parts of he organic acid at room temperature, heating the reaction mass, optionally under pressure, allowing the reaction to proceed, separating the salt from the liquid phase and recovering a reaction product comprising an ester of 5-hydroxymethylfurfural.

The C6 carbohydrate starting material can be selected from mono-(e.g., glucose, galactose, fructose, mannose), di- (e.g., sucrose, lactose, maltose, cellobiose), oligo- (e.g., α-, β- or γ-cyclodextrins) or polysaccharides (e.g., starch, cellulose, inulin), or a mixture thereof, all of which are composed of 6 carbon atom sugar units. Monosaccharides such as glucose and fructose, especially the latter, and disaccharides e.g., sucrose are preferred starting materials for use in the invention.

The organic acid serves a dual function, both of a reaction medium and a reactant (e.g., acetylation reagent, when acetic acid is used). It should be noted that the reaction mass is devoid of anhydrides or acyl halides, which are the most reactive of the carboxylic acid derivatives and are often used in organic synthesis for acetylation. However, in the present case, neither acetic anhydride nor acetyl chloride needs to be added to the reaction vessel to advance the conversion of C6 carbohydrate to AMF. In its most general form, the carboxylic acid is of the formula R-COOH, wherein R is a linear or branched C1-C20 alkyl (e.g., C1-C8, e.g., C1-C5) or linear or branched C2-C20 alkenyl (e.g., C1-C8, e.g., C1-C5). The acids include, e.g., straight-chain saturated or unsaturated carboxylic acids (such as acetic acid, propionic acid, butyric acid, valeric acid, caproic acid, heptanoic acid, octanoic acid, acrylic acid, isocrotonic acid etc.), or branched-chain saturated or unsaturated carboxylic acids (e.g., isobutyric acid, methacrylic acid, etc.), or straight or branched, saturated or unsaturated fatty acids (e.g., myristoleic acid, palmitoleic acid, sapienic acid, oleic acid, etc.). The acid is supplied to the reaction vessel in a pure form, e.g., acetic acid used as anhydrous acetic acid (glacial acetic acid), but in some cases, the presence of water (0-20 wt.%) may have some benefits in promoting the reaction, especially for relatively stable starting materials such as glucose. The reaction vessel is preferably free of added alkyl esters of the carboxylic acid, such as methyl acetate. Although the alkyl esters may form the corresponding acid in situ, the alcohol by- product is generally undesirable (i.e., methanol, in the case of methyl acetate). In case that a mixture of HMF esters is needed, a mixture of carboxylic acids can be used.

Turning now to the salts used to catalyze the reaction, data on the solubility of many salts in anhydrous acetic acid at room temperature can be found in J. Am. Chem. Soc., 50, 7, 1928, 1890-1895 and J. Am. Chem. Soc., 60, 9, 1938, 2043-2046. The salts of choice are preferably alkali metal halides ( e.g., chlorides, bromides and iodides, such as sodium chloride, potassium chloride, sodium bromide, potassium bromide and potassium iodide), ammonium halides (ammonium chloride, ammonium bromide, ammonium iodide), metal bisulfates (such as alkali bisulfates), ammonium bisulfate, ammonium sulfate, and monovalent, divalent and trivalent metal sulfates and their hydrates (alkali metal sulfates, alkaline earth metal sulfates, transition metal or post-transition metal sulfates such as aluminum sulfate and tin sulfate, including double salts consisting of sulfate of a trivalent cation, such as aluminum, water of hydration and sulfate of a monovalent cation, such as potassium, sodium or ammonium, namely, alum compounds, especially KAl(SO₄)₂ and NH₄Al(SO₄)₂, or a mixture consisting of two or more of the salts mentioned above. For example, mixtures of individual salts that are listed above may be selected and proportioned to control the distribution of AMF and HMF. On the other hand, halides of alkaline earth metals and halides of (post) transition metals usually dissolve readily in glacial acetic acid (except for calcium fluoride, CaF2, which can be used in the invention), i.e., more than ten parts in one hundred parts of the acid.

The salts can be used either in a bulk form or in a supported form. Customary solid forms include powders, pellets, flakes, etc. The supports include metal oxides (such as alumina, silica, titanium oxide, zirconium oxide, etc.), clays (montmorillonite K-10, Kaolin, Bentonite, etc.), zeolites, activated carbon, etc.

To carry out the reaction, the C6 carbohydrate, the salt (e.g., halide, bisulfate, or sulfate salt) and the carboxylic acid (e.g., acetic acid) are charged to a reaction vessel. The concentrations of the C6 carbohydrate and the salt in the organic acid are, for example, in the ranges from 0.01 g/ml to 0.4 g/ml and 0.005 g/ml to 0.2 g/ml, respectively.

The reaction is carried out under heating, e.g., at a temperature of not less than 80 °C, e.g., >100 °C. The reaction temperature depends on the reagents (the salt catalyst, carboxylic acid, and C₆ carbohydrate). For example, the temperature range for the reaction of fructose with acetic acid in the presence of alkali halide salts as a catalyst is from 120 to 200 °C, preferably from 140 to 200 °C and more preferably from 140 to 180 °C. The temperature range for the reaction of fructose with acetic acid in the presence of ammonium chloride, NH₄Cl, as a catalyst is from 100 to 200 °C, preferably from 110 to 180 °C and more preferably 110-150 °C.

The reaction can proceed under atmospheric pressure when a relatively low temperature is applied, e.g., under reflux, or with heating in a sealed reactor under autogenous pressure. The reaction vessel may also be pressurized with inert gas to 1-30 bar, when the temperature is considerably higher than the reflux temperature of carboxylic acid used if it is desired to keep the solvent (the organic acid) in a liquid state. Thus, the reaction vessel is usually a high-pressure reactor, a batch (e.g., borosilicate ACE pressure tube, Hastelloy coated Parr batch reactor, etc.) or a flow reactor; hydrogenator or steel autoclave type reactors can be used. However, in the presence of bisulfate, either as a sole catalyst or in combination with other salts, a reaction conducted under reflux was shown to be very efficient.

On a laboratory scale, the reaction lasts up to five hours (depending on catalyst type and temperature). The progress of the reaction can be monitored by customary analytical techniques (e.g., TLC, HPLC, etc.). The monitoring is performed to determine the conversion of the starting carbohydrate and the yields and ratio of targeted HMF and HMF ester(s).

Chloride salts that were tested in the experimental part reported below to convert fructose (NaCl and NH₄Cl), gave both fairly good results. The reactions afforded AMF:HMF product mixture proportioned ~3:2 (by yield, determined by HPLC, with NH₄Cl outperforming NaCl in terms of reaction time (2 hours versus 3 hours) and temperature (130 °C versus 150 °C). Conversion of fructose was also advanced efficiently in acetic acid at 150 °C using a sulfate salt, e.g., alum, affording AMF:HMF product mixture proportioned ~3:1 (by yield, determined by HPLC).

Conversion of carbohydrates in acetic acid was advanced efficiently under reflux (acetic acid boiling point is 118 °C), in the presence of alkali bisulfate (e.g., NaHSO₄·H₂O) affording AMF:HMF product mixture proportioned >5:1, e.g., ~7:1 (by yield, determined by HPLC).

Thus, a specific aspect of the invention is a process comprising adding to a reactor (e.g., a pressure reactor) fructose, a catalyst selected from the group consisting of alkali halides, metal sulfates, metal and ammonium bisulfates; and acetic acid, optionally sealing and pressurizing the reactor, heating the reaction mass to a temperature in the range from 100 to 200°C, e.g., 140 to 200°C, allowing the reaction to proceed, cooling the reaction mass and working-up the reaction mass to obtain a solution comprising a mixture consisting of AMF and HMF, or AMF alone. AMF is the predominant component of said mixture. For example, with metal sulfate such as potassium aluminum sulfate, the ratio AMF:HMF was above 2.5:1.

Another specific aspect of the invention is a process comprising adding to a reactor (e.g., a pressure reactor) fructose, ammonium halide (e.g., chloride or bromide) and acetic acid, optionally sealing and pressurizing the reactor, heating the reaction mass to a temperature in the range from 100 to 200°C, e.g., from 100 to 140°C, allowing the reaction to proceed, cooling the reaction mass and working-up the reaction mass to obtain a solution comprising a mixture consisting of AMF and HMF. AMF is the predominant component of said mixture.

We have also tested the conversion of sucrose in acetic acid (optionally with up to 20% v/v water), in the presence of alkali halides (e.g., sodium chloride, potassium chloride and potassium bromide), alkali bisulfate (e.g., potassium bisulfate), ammonium bisulfate and sulfate salts of aluminum, namely, Al₂ (SO₄)₃·18H₂O and also alum salts (e.g., NH₄Al(SO₄)₂·12H₂O) over the temperature range from 80 to 200°C, e.g., 100 to 200°C, for example, from 120 to 180°C, and found that AMF is obtained as the predominant reaction product over HMF.

Thus, another specific aspect of the invention is a process comprising adding to a reactor (e.g., a pressure reactor) sucrose; a catalyst selected from the group consisting of alkali halides, metal sulfates, alkali metal bisulfate or ammonium bisulfate; and acetic acid, optionally sealing and pressurizing the reactor, heating the reaction mass to a temperature in the range from 100 to 200°C, e.g., 120 to 180°C, allowing the reaction to proceed, cooling the reaction mass and working-up the reaction mass to obtain a solution comprising a mixture consisting of AMF and HMF (AMF is the predominant component of said mixture), or AMF alone. For example, with ammonium bisulfate, sucrose was converted exclusively to AMF.

Experimental work shown below indicates that bisulfates, such as alkali bisulfate, are also effective when the reaction is run under atmospheric pressure. Another specific aspect of the invention is a process comprising combining in a reaction vessel a carbohydrate (such as fructose), a bisulfate salt (such as alkali bisulfate) and acetic acid, heating the reaction mass to temperature in the range from 80 to reflux (e.g., 118 °C), allowing the reaction to proceed (reaction times are fairly reasonable), cooling the reaction mass and working-up the reaction mass to obtain a solution comprising a mixture consisting of AMF and HMF (AMF is the predominant component of said mixture), or AMF alone.

For terminating the reaction, the reaction vessel is cooled down to room temperature (and, when an autoclave or the like has been used, pressure is released), and the reaction mass is worked-up. As pointed out above, owing to their low solubility in the organic acid, the salt catalysts can be separated at the end of the reaction, by conventional solid/liquid separation techniques such as filtration, decantation, etc., and recycled. For example, after filtration the salt is washed with acetic acid and, if additional washing is necessary, subjected to washing with acetone. The acidic filtrate may be treated with an anti-solvent, e.g., an ether such as methyl tertbutyl ether, to precipitate humic materials, which are removed by filtration, affording a solution with higher purity of the products HMF ester and HMF in the organic acid.

For some applications, the mixture of products might be used without separating HMF ester(s) from the HMF, while if desired, HMF ester(s), e.g., AMF, can be separated by extraction or vacuum distillation (see for example E.S. Kang et al., "From lignocellulosic biomass to furans via 5-acetoxymethylfurfural as an alternative to 5-hydroxymethylfurfural", ChemSusChem, 8, 2015, 1179-1188). At the end of the reaction, the carboxylic acid, e.g., acetic acid, can be separated and recycled by evaporation. Fractional distillation is optional if recovery of, e.g., pure acetic acid is desired. Thus, the invention enables the recovery of the carboxylic acid and salt catalyst that are used in the process. As pointed out above, AMF, in addition to being a useful product in its own right, is an important chemical intermediate that can be used in the synthesis of some beneficial end-products. Non-limiting examples of useful chemicals that may be synthesized from AMF are shown below:

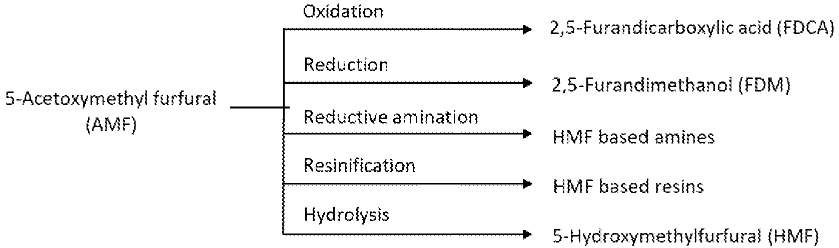

2,5-Furandimethanol (FDM) is obtained by reducing AMF, e.g., with sodium borohydride, molecular hydrogen or other reducing agents. For example, sodium borohydride is added to a solution of AMF in methanol at room temperature. The reaction mixture is stirred and then concentrated. The crude product is dissolved in water and neutralized to pH 6-7. Then the solution is extracted with an organic solvent, dried and concentrated under reduced pressure. FDM can be recovered from the crude by column chromatography.

Tertiary amine derivatives of furfuryl alcohol (that is, the group -CH₂-N(Alk)₂ is attached at position 5 of the ring, for example, 5-(diethylaminomethyl) furfuryl alcohol, 5-(dimethylaminomethyl) furfuryl alcohol and acid addition salts thereof; Alk is independently C1-C3 alkyl) are prepared by reductive amination, namely, reacting AMF with dialkylamine, such as diethyl amine or dimethyl amine. For example, anhydrous sodium sulfate is added to a solution of AMF in methanol followed by addition of diethylamine. The reaction mixture is stirred for a few fours (e.g., 2 hours) and sodium borohydride is added at room temperature with stirring for another 18 hours. Then, the resulting solution is filtrated, washed with methanol, and evaporated to obtain a viscous residue. The residue is separated between chloroform and 30% sodium hydroxide aqueous solution. The organic phase is dried over anhydrous magnesium sulfate and evaporated to afford the tertiary amine product.

Because AMF is more efficiently separable from the reaction mixture compared to HMF, it is also beneficial to employ the present invention to produce the readily separable AMF as the major reaction product, separate the AMF, and then hydrolyze AMF to HMF. AMF is selectively transformed to HMF with high yield and purity through a base-catalyzed hydrolysis (e.g., using NaOH, K₂CO₃ or a strongly basic ion-exchange resin such as Amberlite IRN78 hydroxide resin, Amberlyst A-26 OH, etc.). For example, the reaction of a solution of AMF in methanol in the presence of potassium carbonate is performed at room temperature for 15 hours. The reaction mixture is filtrated and evaporated. The crude is diluted with ethyl acetate, washed with brine, dried and evaporated to afford crude HMF (S. Shinde, K. Deval, R. Chikate, C. Rode, "Cascade synthesis of 5-(acetoxymethyl)furfural from carbohydrates over Sn-Mont catalyst", ChemistrySelect, 3, 2018, 8770-8778).

Accordingly, the invention further comprises the step of isolating AMF from the solution of AMF and HMF, optionally purifying the AMF, and a further step of:
oxidation of AMF to 2,5-Furandicarboxylic acid; reduction of AMF to 2,5-Furandimethanol;
reductive amination of AMF in the presence of dialkylamine to form a tertiary amine derivative of furfuryl alcohol; or hydrolysis of AMF to HMF.

In its turn, HMF obtained from AMF can be converted into valuable products via one or more successive reaction steps. Non-limiting examples are depicted below:

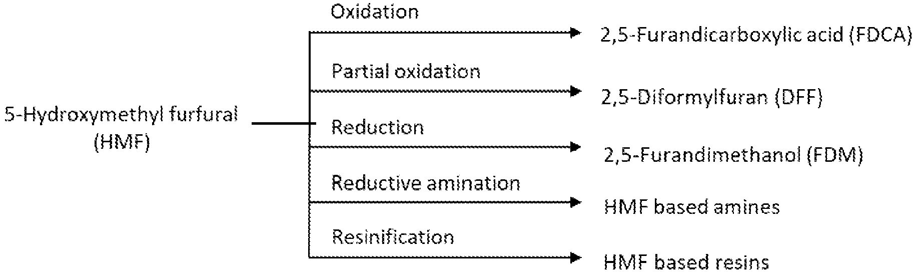

Selective oxidation of HMF to 2,5-Diformylfuran (DFF) can be performed in the presence of many types of catalysts, for example, manganese oxide-based catalysts, GO-related catalysts, supported Ru catalysts, and vanadium-based catalysts, e.g., vanadium phosphate oxide (VPO). For example, a solution of HMF in DMSO in the presence of vanadium phosphate oxide catalyst is heated and stirred at 120 °C for 10 hours under air atmosphere to afford some substances with DFF as the major product (J. Lai et al., "Selective oxidation of 5-hydroxymethylfurfural into 2,5-diformylfuran over VPO catalysts under atmospheric pressure", RSC Adv., 9, 2019, 14242-14246).

Resinification of HMF is performed with an amine (e.g., urea, melamine, etc.) or phenolic compound. (e.g., phenol, cresol, hydroquinone, etc.). Resins formation can be catalyzed with an alkaline catalyst (e.g., NaOH, KOH, K₂CO₃, etc.), an acid catalyst (e.g., H₂SO₄, HCl, oxalic acid, acetic acid, etc.) or by a combination of both. For example, a mixture of phenol, HMF and 50% sodium hydroxide solution catalyst is heated to 110 °C and stirred for an appropriate period to completion (analytical monitoring). The reaction mixture is cooled to 80 °C and evaporated under a vacuum to remove water and unreacted phenol. An anti-solvent precipitation by addition of methanol/water, followed by filtration and washing the solid product with deionized water affords HMF-phenol resin (US 9725552).

Other reactions starting from HMF in the scheme depicted above are performed in a manner similar to that previously described for AMF.

One important chemical that is prepared either directly from AMF, or from HMF that was obtained from AMF, is 2,5-Furandicarboxylic acid (FDCA), a monomer needed to make the polymer polyethylene furanoate (PEF), which is recognized as an important bioplastic. That is, FDCA prepared as described above is coupled with ethylene glycol to form PEF.

### Examples

HPLC method conditions: chromatographic analyses were carried out with Agilent HPLC 1200 series. Column: Eclipse XDB C18 (4.6×150 mm); Injection volume capacity: 5µl; Column temperature: 35 °C; Mobile phase A: 0.1% formic acid in water; Mobile Phase B: methanol; Mobile phase consisted of A:B mixture: 80:20; Flow rate: 0.8 mL/min; Detection was performed at 280 nm; Injection volume: 1 µL; Run time: 17 minutes.

### Example 1

### Preparation of AMF from Fructose in the presence of NaCl

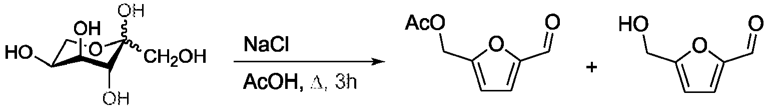

A pressure tube equipped with a magnetic stirrer was charged with fructose (1.06g) and sodium chloride (0.75g), followed by glacial Acetic acid (10 ml). The pressure tube was sealed, heated to 150 °C (oil bath) and stirred for 3h. Then, the reaction flask was cooled to room temperature, the reaction mixture was filtrated, to remove the salt, which was washed with acetic acid. MTBE (60 ml) was added under stirring to the acidic filtrate. The resulting solution was filtered, washed with MTBE and distilled by vacuum (to remove MTBE). AMF and HMF yields determined by HPLC: 29.48% and 21.87%, respectively.

### Example 2

### Preparation of AMF from Fructose in the presence of NH₄Cl

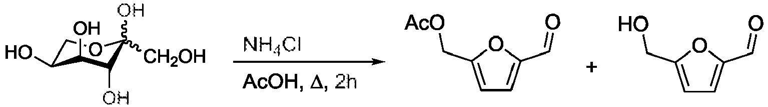

A pressure tube equipped with a magnetic stirrer was charged with fructose (1.00g) and ammonium chloride (0.50g), followed by glacial acetic acid (10 ml). The pressure tube was sealed, heated to 130 °C (oil bath) and stirred for 2h. Then, the reaction flask was cooled to room temperature, the reaction mixture was filtrated, to remove the salt, which was washed with acetic acid. MTBE (60 ml) was added under stirring to the acidic filtrate. The resulting solution was filtered, washed with MTBE and distilled by vacuum (to remove MTBE). AMF and HMF yields determined by HPLC: 24.26% and 10.99%, respectively.

### Example 3

### Preparation of AMF from Fructose in the presence of potassium aluminum sulfate dodecahydrate

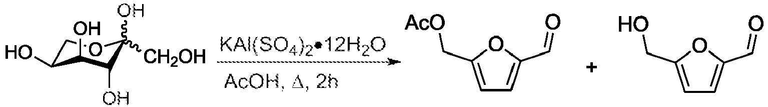

A pressure tube equipped with a magnetic stirrer was charged with fructose (1.00g) and potassium aluminum sulfate dodecahydrate (0.50g), followed by glacial acetic acid (10 ml). The pressure tube was sealed, heated to 150 °C (oil bath) and stirred for 2h. Then, the reaction flask was cooled to room temperature, the reaction mixture was filtrated, to remove the salt, which was washed with acetic acid. MTBE (60 ml) was added under stirring to the acidic filtrate. The resulting solution was filtered, washed with MTBE and distilled by vacuum (to remove MTBE). AMF and HMF yields determined by HPLC: 31.46% and 11.18%, respectively.

### Example 4

### Preparation of AMF from fructose in the presence of sodium bisulfate monohydrate

A round bottom flask equipped with a magnetic stirrer and connected to a condenser was charged with fructose (1.00g) and sodium hydrogen sulfate monohydrate (0.75g), followed by glacial acetic acid (10 ml). The flask was heated to reflux (oil bath) and stirred for 1h. Then, the reaction flask was cooled to room temperature, the reaction mixture was filtrated, to remove the salt, which was washed with acetic acid. MTBE (60 ml) was added under stirring to the acidic filtrate. The resulting solution was filtered, washed with MTBE and distilled by vacuum (to remove MTBE). AMF and HMF yields determined by HPLC: 29.18% and 3.81%, respectively.

### Examples 5-11

### Preparation of AMF from Sucrose

The procedure of Example 3 was performed with sucrose (1.0g) at 150 °C. The reaction conditions (salt catalyst, solvent, reaction time) and yields of AMF and HMF are shown in Table 1.

**Table 1.**

| Ex. | Catalyst | Solvent | Reaction time, min | Yield AMF, % | Yield HMF, % |
|---|---|---|---|---|---|
| 5 | Ammonium aluminum sulfate dodecahydrate, NH₄Al(SO₄)₂·12H₂O, 0.75g | Acetic acid, 10 ml | 120 | 21.71 | 9.38 |
| 6 | Aluminum sulfate octadecahydrate, Al₂(SO₄)₃·18H₂O, 0.75g | Acetic acid, 10 ml | 120 | 24.72 | 7.53 |
| 7 | Potassium bisulfate, KHSO₄, 0.75g | Acetic acid, 10 ml | 120 | 22.16 | 8.25 |
| 8 | Ammonium bisulfate, NH₄HSO₄, 0.75g | Acetic acid, 10 ml | 120 | 22.5 | 0 |
| 9 | Potassium chloride, KCl, 1g | Acetic acid, 9 ml + H₂O, 1ml | 180 | 23.29 | 17.10 |
| 10 | Sodium chloride, NaCl, 1g | Acetic acid, 9 ml + H₂O, 1ml | 180 | 26.94 | 18.86 |
| 11 | Potassium bromide, KBr, 1g | Acetic acid, 10 ml | 180 | 34.78 | 19.76 |

## Claims

1. A process for preparing an ester of 5-hydroxymethylfurfural of the formula: 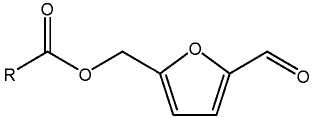 wherein R is a linear or branched alkyl or alkenyl, comprising adding to a reaction vessel C₆ carbohydrate, organic acid R-COOH and a salt which is at most sparingly soluble in said organic acid to form a reaction mass, wherein the salt is selected such that not more than three parts of the salt are dissolved in one hundred parts of the organic acid at room temperature, heating the reaction mass, allowing the reaction to proceed, separating the salt from the liquid phase and recovering a reaction product comprising an ester of 5-hydroxymethylfurfural.

2. The process according to claim 1, wherein the organic acid added is acetic acid, such that the ester formed is 5-acetoxymethyl furfural of the formula:

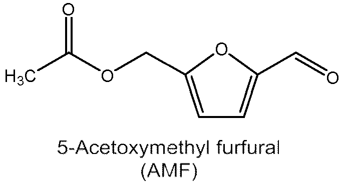

3. The process according to claim 1 or 2, wherein the C₆ carbohydrate is a monosaccharide or disaccharide.

4. The process according to claim 3, wherein: (i) the monosaccharide is fructose; (ii) the disaccharide is sucrose.

5. The process according to any one of claims 1 to 4, wherein the salt is selected from the group consisting of:
alkali metal halides;
ammonium halides;
metal bisulfates;
ammonium bisulfate;
ammonium sulfate; and
monovalent, divalent and trivalent metal sulfates and their hydrates, including double salts consisting of sulfate of a trivalent cation, water of hydration and sulfate of a monovalent cation; and
mixtures consisting of two or more of said salts.

6. The process according to claim 5, wherein the salt is selected from the group consisting of sodium chloride, potassium chloride, potassium bromide, ammonium chloride, potassium aluminum sulfate dodecahydrate, ammonium aluminum sulfate dodecahydrate, aluminum sulfate octadecahydrate, potassium bisulfate, sodium bisulfate and ammonium bisulfate.

7. The process according to any one of claims 1 to 6, wherein the reaction product is recovered by cooling the reaction mass, preferably to room temperature at the end of the reaction, separating the salt from the liquid phase, to obtain a solution comprising a mixture consisting of the ester of 5-hydroxymethylfurfural and 5-hydroxymethylfurfural, or the ester alone.

8. The process according to any one of claims 1 to 6, comprising adding to a reactor fructose; a catalyst selected from the group consisting of alkali halides, metal sulfates, metal bisulfates and ammonium bisulfate; and acetic acid, optionally sealing and pressurizing the reactor, heating the reaction mass to a temperature in the range from 140 to 200°C, allowing the reaction to proceed, cooling the reaction mass and working-up the reaction mass to obtain a solution comprising a mixture consisting of AMF and HMF, wherein AMF is the predominant component of said mixture, or AMF alone.

9. The process according to any one of claims 1 to 6, comprising adding to a reactor fructose, ammonium halide and acetic acid, optionally sealing and pressurizing the reactor, heating the reaction mass to a temperature in the range from 100 to 140°C, allowing the reaction to proceed, cooling the reaction mass and working-up the reaction mass to obtain a solution comprising a mixture consisting of AMF and HMF, wherein AMF is the predominant component of said mixture, or AMF alone.

10. The process according to any one of claims 1 to 6, comprising adding to a reactor sucrose; a catalyst selected from the group consisting of alkali halides, metal sulfates, alkali metal bisulfate and ammonium bisulfate; and acetic acid, optionally sealing and pressurizing the reactor, heating the reaction mass to a temperature in the range from 100 to 200 °C, allowing the reaction to proceed, cooling the reaction mass and working-up the reaction mass to obtain a solution comprising a mixture consisting of AMF and HMF, wherein AMF is the predominant component of said mixture, or AMF alone.

11. The process according to any one of claims 1 to 6, comprising combining in a reaction vessel the carbohydrate, a bisulfate salt and acetic acid, heating the reaction mass to a temperature in the range from 80 °C to reflux, allowing the reaction to proceed, cooling the reaction mass and working-up the reaction mass to obtain a solution comprising a mixture consisting of AMF and HMF, or AMF alone.

12. The process according to any one of claims 1 to 11, wherein the reaction mass is devoid of anhydrides or acyl halides.

13. The process according to any one of claims 2 to 12, further comprising the step of isolating AMF from the solution of AMF and HMF, and optionally purifying the AMF.

14. The process according to claim 13, further comprising a step of:
oxidation of AMF to 2,5-Furandicarboxylic acid; reduction of AMF to 2,5-Furandimethanol; reductive amination of AMF in the presence of dialkylamine to form a tertiary amine derivative of furfuryl alcohol; or hydrolysis of AMF to HMF.

15. The process according to claim 14, wherein AMF is hydrolyzed to HMF, the process further comprises a step of:
oxidation of the so-formed HMF to 2,5-Furandicarboxylic acid;
oxidation of the so-formed HMF to 2,5-Diformylfuran;
reduction of the so-formed HMF to 2,5-Furandimethanol;
reductive amination of the so-formed HMF; or resinification of HMF with an amine or aromatic compound.

## Patentansprüche

1. Verfahren zur Herstellung eines Esters von 5-Hydroxymethylfurfural der Formel: 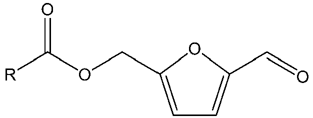 wobei R ein linearer oder verzweigter Alkyl- oder Alkenylrest ist, umfassend das Einbringen von einem C6-Kohlenhydrat, einer organischen Säure R-COOH und einem Salz, das in dieser organischen Säure höchstens schwer löslich ist, in ein Reaktionsgefäß, um eine Reaktionsmasse zu bilden, wobei das Salz so ausgewählt ist, dass bei Raumtemperatur nicht mehr als drei Teile des Salzes in hundert Teilen der organischen Säure gelöst sind, das Erhitzen der Reaktionsmasse, das Ermöglichen des Vonstattengehens der Reaktion, das Abtrennen des Salzes aus der flüssigen Phase und das Gewinnen eines Reaktionsprodukts, welches einen Ester von 5-Hydroxymethylfurfural umfasst.

2. Das Verfahren nach Anspruch 1, wobei die zugesetzte organische Säure Essigsäure ist, sodass der gebildete Ester 5-Acetoxymethylfurfural der folgenden Formel ist:

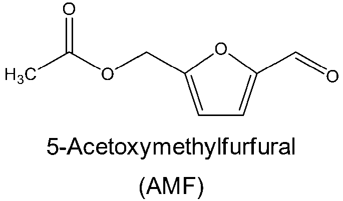

3. Das Verfahren nach Anspruch 1 oder 2, wobei das C6-Kohlenhydrat ein Monosaccharid oder Disaccharid ist.

4. Das Verfahren nach Anspruch 3, wobei: (i) das Monosaccharid Fructose ist; (ii) das Disaccharid Saccharose ist.

5. Das Verfahren nach einem der Ansprüche 1 bis 4, wobei das Salz aus der Gruppe ausgewählt ist, bestehend aus:
Alkalimetallhalogeniden;
Ammoniumhalogeniden;
Metallhydrogensulfaten;
Ammoniumhydrogensulfat;
Ammoniumsulfat; und
Sulfaten von ein-, zwei- und dreiwertigen Metallen sowie deren Hydraten, einschließlich Doppelsalzen, die aus dem Sulfat eines dreiwertigen Kations, Hydratwasser und dem Sulfat eines einwertigen Kations bestehen; und
Mischungen, die aus zwei oder mehr der genannten Salze bestehen.

6. Das Verfahren nach Anspruch 5, wobei das Salz aus der Gruppe ausgewählt ist, bestehend aus: Natriumchlorid, Kaliumchlorid, Kaliumbromid, Ammoniumchlorid, Kaliumaluminiumsulfat-Dodecahydrat, Ammoniumaluminiumsulfat-Dodecahydrat, Aluminiumsulfat-Octadecahydrat, Kaliumhydrogensulfat, Natriumhydrogensulfat und Ammoniumhydrogensulfat.

7. Das Verfahren nach einem der Ansprüche 1 bis 6, wobei das Reaktionsprodukt gewonnen wird, indem die Reaktionsmasse abgekühlt wird, vorzugsweise auf Raumtemperatur am Ende der Reaktion, und das Salz aus der flüssigen Phase abgetrennt wird, um eine Lösung zu erhalten, umfassend: eine Mischung, bestehend aus dem Ester von 5-Hydroxymethylfurfural und 5-Hydroxymethylfurfural, oder den Ester allein.

8. Das Verfahren nach einem der Ansprüche 1 bis 6, umfassend die Zugabe von Fructose; einem Katalysator, der aus der Gruppe, bestehend aus Alkalihalogeniden, Metallsulfaten, Metallhydrogensulfaten und Ammoniumhydrogensulfat, ausgewählt ist; und Essigsäure in einen Reaktor, gegebenenfalls das Verschließen und mit Druck beaufschlagen des Reaktors, das Erhitzen der Reaktionsmasse auf eine Temperatur im Bereich von 140 bis 200 °C, das Ermöglichen des Vonstattengehens der Reaktion, das Abkühlen der Reaktionsmasse und Aufarbeiten der Reaktionsmasse, um eine Lösung zu erhalten, umfassend: eine Mischung, bestehend aus AMF und HMF, wobei AMF die Hauptkomponente dieser Mischung ist, oder AMF allein.

9. Das Verfahren nach einem der Ansprüche 1 bis 6, umfassend die Zugabe von Fructose, Ammoniumhalogenid und Essigsäure in einen Reaktor, gegebenenfalls das Verschließen und mit Druck beaufschlagen des Reaktors, das Erhitzen der Reaktionsmasse auf eine Temperatur im Bereich von 100 bis 140 °C, das Ermöglichen des Vonstattengehens der Reaktion, das Abkühlen der Reaktionsmasse und Aufarbeiten der Reaktionsmasse, um eine Lösung zu erhalten, umfassend: eine Mischung bestehend aus AMF und HMF, wobei AMF die Hauptkomponente dieser Mischung ist, oder AMF allein.

10. Das Verfahren nach einem der Ansprüche 1 bis 6, umfassend die Zugabe von Saccharose; einem Katalysator, der aus der Gruppe, bestehend aus Alkalihalogeniden, Metallsulfaten, Alkalimetallhydrogensulfaten und Ammoniumhydrogensulfat, ausgewählt ist; und Essigsäure in einen Reaktor, gegebenenfalls das Verschließen und mit Druck beaufschlagen des Reaktors, das Erhitzen der Reaktionsmasse auf eine Temperatur im Bereich von 100 bis 200 °C, das Ermöglichen des Vonstattengehens der Reaktion, das Abkühlen der Reaktionsmasse und Aufarbeiten der Reaktionsmasse, um eine Lösung zu erhalten, umfassend: eine Mischung, bestehend aus AMF und HMF, wobei AMF die Hauptkomponente dieser Mischung ist, oder AMF allein.

11. Das Verfahren nach einem der Ansprüche 1 bis 6, umfassend das in Verbindung bringen des Kohlenhydrats, eines Bisulfatsalzes und der Essigsäure in einem Reaktionsgefäß, das Erhitzen der Reaktionsmasse auf eine Temperatur im Bereich von 80 °C bis zur Rückflusstemperatur, das Ermöglichen des Vonstattengehens der Reaktion, das Abkühlen der Reaktionsmasse und Aufarbeiten der Reaktionsmasse, um eine Lösung zu erhalten, umfassend: eine Mischung, bestehend aus AMF und HMF, oder AMF allein.

12. Das Verfahren nach einem der Ansprüche 1 bis 11, wobei die Reaktionsmasse frei von Anhydriden oder Acylhalogeniden ist.

13. Das Verfahren nach einem der Ansprüche 2 bis 12, ferner umfassend den Schritt des Abtrennens von AMF aus der Lösung von AMF und HMF, und, optional, des Reinigens von AMF.

14. Das Verfahren nach Anspruch 13, ferner umfassend einen Schritt: der Oxidation von AMF zu 2,5-Furandicarbonsäure; der Reduktion von AMF zu 2,5-Furandimethanol; der reduktiven Aminierung von AMF in Gegenwart von Dialkylamin zur Bildung eines tertiären Aminderivats von Furfurylalkohol; oder der Hydrolyse von AMF zu HMF.

15. Das Verfahren nach Anspruch 14, wobei AMF zu HMF hydrolysiert wird und das Verfahren ferner umfasst: einen Schritt der Oxidation des so gebildeten HMF zu 2,5-Furandicarbonsäure; einen Schritt der Oxidation des so gebildeten HMF zu 2,5-Diformylfuran; einen Schritt der Reduktion des so gebildeten HMF zu 2,5-Furandimethanol; einen Schritt der reduktiven Aminierung des so gebildeten HMF; oder einen Schritt der Harzbildung von HMF mit einem Amin oder einer aromatischen Verbindung.

## Revendications

1. Procédé de préparation d'un ester de 5-hydroxyméthylfurfural de formule: 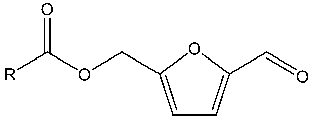 dans laquelle R représente un groupe alkyle ou alcényle linéaire ou ramifié, comprenant l'ajout, dans un réacteur, d'un glucide en C6, d'un acide organique R-COOH et d'un sel qui est au plus faiblement soluble dans ledit acide organique pour former une masse réactionnelle, le sel étant choisi de telle sorte qu'au plus trois parties du sel se dissolvent dans cent parties de l'acide organique à température ambiante, le chauffage de la masse réactionnelle, permettant la réaction se dérouler, la séparation du sel de la phase liquide et la récupération d'un produit réactionnel comprenant un ester de 5-hydroxyméthylfurfural.

2. Procédé selon la revendication 1, dans lequel l'acide organique ajouté est l'acide acétique, de sorte que l'ester formé est le 5-acétoxyméthylfurfural de formule:

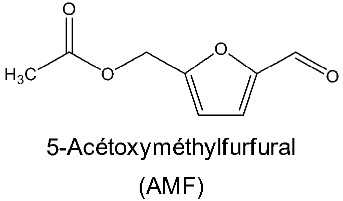

3. Procédé selon la revendication 1 ou 2, dans lequel le glucide en C6 est un monosaccharide ou un disaccharide.

4. Procédé selon la revendication 3, dans lequel: (i) le monosaccharide est le fructose; (ii) le disaccharide est le saccharose.

5. Procédé selon l'une quelconque des revendications 1 à 4, dans lequel le sel est choisi dans le groupe constitué par:
les halogénures de métaux alcalins;
les halogénures d'ammonium;
les bisulfates métalliques;
le bisulfate d'ammonium;
le sulfate d'ammonium; et
les sulfates de métaux monovalents, divalents et trivalents ainsi que leurs hydrates, y compris les sels doubles constitués d'un sulfate de cation trivalent, d'eau d'hydratation et d'un sulfate de cation monovalent; et
les mélanges constitués de deux ou plusieurs desdits sels.

6. Procédé selon la revendication 5, dans lequel le sel est choisi dans le groupe constitué par le chlorure de sodium, le chlorure de potassium, le bromure de potassium, le chlorure d'ammonium, le sulfate d'aluminium et de potassium dodécahydraté, le sulfate d'aluminium et d'ammonium dodécahydraté, le sulfate d'aluminium octadécahydraté, le bisulfate de potassium, le bisulfate de sodium et le bisulfate d'ammonium.

7. Procédé selon l'une quelconque des revendications 1 à 6, dans lequel le produit de réaction est récupéré par refroidissement de la masse réactionnelle, de préférence jusqu'à la température ambiante à la fin de la réaction, et séparation du sel de la phase liquide, pour obtenir une solution comprenant un mélange constitué de l'ester du 5-hydroxyméthylfurfural et du 5-hydroxyméthylfurfural, ou de l'ester seul.

8. Procédé selon l'une quelconque des revendications 1 à 6, comprenant l'ajout dans un réacteur de fructose; d'un catalyseur choisi dans le groupe constitué par les halogénures de métaux alcalins, les sulfates métalliques, les bisulfates métalliques et le bisulfate d'ammonium; et d'acide acétique, le cas échéant la fermeture hermétique et la mise sous pression du réacteur, le chauffage de la masse réactionnelle à une température comprise entre 140 et 200 °C, permettant la réaction se dérouler, le refroidissement de la masse réactionnelle et le traitement de la masse réactionnelle pour obtenir une solution comprenant un mélange constitué d'AMF et de HMF, où l'AMF est le constituant prédominant dudit mélange, ou de l'AMF seul.

9. Procédé selon l'une quelconque des revendications 1 à 6, comprenant l'ajout dans un réacteur de fructose, d'un halogénure d'ammonium et d'acide acétique, le cas échéant la fermeture hermétique et la mise sous pression du réacteur, le chauffage de la masse réactionnelle à une température comprise entre 100 et 140 °C, permettant la réaction se dérouler, le refroidissement de la masse réactionnelle et le traitement de la masse réactionnelle pour obtenir une solution comprenant un mélange constitué d'AMF et de HMF, où l'AMF est le constituant prédominant dudit mélange, ou de l'AMF seul.

10. Procédé selon l'une quelconque des revendications 1 à 6, comprenant l'ajout dans un réacteur de saccharose; d'un catalyseur choisi dans le groupe constitué par les halogénures de métaux alcalins, les sulfates métalliques, les bisulfates de métaux alcalins et le bisulfate d'ammonium; et d'acide acétique, le cas échéant la fermeture hermétique et la mise sous pression du réacteur, le chauffage de la masse réactionnelle à une température comprise entre 100 et 200 °C, permettant la réaction se dérouler, le refroidissement de la masse réactionnelle et le traitement de la masse réactionnelle pour obtenir une solution comprenant un mélange constitué d'AMF et de HMF, où l'AMF est le constituant prédominant dudit mélange, ou de l'AMF seul.

11. Procédé selon l'une quelconque des revendications 1 à 6, comprenant la combinaison, dans un réacteur, du glucide, d'un sel de bisulfate et d'acide acétique, le chauffage de la masse réactionnelle à une température comprise entre 80 °C et le reflux, permettant la réaction se dérouler, le refroidissement de la masse réactionnelle et le traitement de la masse réactionnelle pour obtenir une solution comprenant un mélange constitué d'AMF et de HMF, ou d'AMF seul.

12. Procédé selon l'une quelconque des revendications 1 à 11, dans lequel la masse réactionnelle est exempt d'anhydrides ou d'halogénures d'acyle.

13. Procédé selon l'une quelconque des revendications 2 à 12, comprenant en outre l'étape consistant à isoler l'AMF de la solution d'AMF et de HMF, et éventuellement à purifier l'AMF.

14. Procédé selon la revendication 13, comprenant en outre une étape de: oxydation de l'AMF en acide 2,5-furanedicarboxylique; réduction de l'AMF en 2,5-furanediméthanol; amination réductrice de l'AMF en présence d'une dialkylamine pour former un dérivé amine tertiaire de l'alcool furfurylique; ou hydrolyse de l'AMF en HMF.

15. Procédé selon la revendication 14, dans lequel l'AMF est hydrolysé en HMF, le procédé comprenant en outre une étape de: oxydation du HMF ainsi formé en acide 2,5-furanedicarboxylique; oxydation du HMF ainsi formé en 2,5-diformylfurane; réduction du HMF ainsi formé en 2,5-furanediméthanol; amination réductrice du HMF ainsi formé; ou résinification du HMF avec une amine ou un composé aromatique.
